# EUROPEAN PATENT SPECIFICATION

(11) **EP 2 110 084 B1**
(45) Date of publication and mention of the grant of the patent: **12.07.2017**
(21) Application number: 09251067.6
(22) Date of filing: 09.04.2009
(51) Int. Cl.: A61B 17/072, A61B 17/10, A61B 17/115

(54) **Single loop surgical fastener apparatus for applying variable compression**
Chirurgische Befestigungsvorrichtung mit einzelner Schlaufe zur Anwendung einer variablen Kompression
Appareil de fixation chirurgicale à boucle unique pour appliquer une compression variable

(30) Priority: 14.04.2008 US 44673 P; 03.04.2009 US 417711
(43) Date of publication of application: 21.10.2009
(73) Proprietor: Covidien LP, Mansfield, MA 02048 (US)
(72) Inventor: Viola, Frank J., Sandy Hook, CT 06482 (US)
(74) Representative: Soames, Candida Jane

(56) References cited:
- US-A1- 2007 131 732
- US-A1- 2007 262 116
- US-A1- 2008 078 804

## Description

### BACKGROUND

### 1. Technical Field

The present disclosure relates to surgical fastener applying apparatus. More particularly, the present disclosure relates to a surgical fastener applying apparatus to apply a plurality of surgical fasteners to tissue with varying compressive forces.

### 2. Background of the Related Art

Many varieties of surgical fastening apparatus are known in the art, some of which are specifically adapted for use in various surgical procedures including, but not limited to, end-to-end anastomosis, circular end-to-end anastomosis, open gastrointestinal anastomosis, endoscopic gastrointestinal anastomosis, and transverse anastomosis. Suitable examples of apparatus which may be used during the course of these procedures can be seen in U.S. Pat. Nos. 5,915,616; 6,202,914; 5,865,361; and 5,964,394.

A further apparatus capable of producing staples of different formed length is disclosed in US 2008/0078804 A1.

In general, a surgical fastening apparatus will include an anvil that is approximated relative to a fastener cartridge during use or a fastener cartridge that is approximated relative to an anvil. The anvil includes depressions that are aligned with, and/or are in registration with slots defined in the cartridge, through which the fasteners will emerge, to effectuate formation. The fastener cartridge typically has one or more rows of fasteners disposed laterally outward of a channel or knife slot that is configured to accommodate a knife, or other such cutting element, such that tissue can be simultaneously cut and joined together. Depending upon the particular surgical fastening apparatus, the rows of fasteners may be arranged in a linear or non-linear, e.g. circular, semi-circular, or otherwise arcuate configuration.

Various types of surgical fasteners are well known in the art, including but not limited to unitary fasteners and two-part fasteners. Unitary fasteners generally include a pair of legs adapted to penetrate tissue and connected by a backspan from which they extend. In use, subsequent to formation, some of the unitary fasteners have a "B" configuration. Typically, the two-part fastener includes legs that are barbed and connected by a backspan which are engaged and locked into a separate retainer piece that is usually located in the anvil. In use, the two-part fastener is pressed into the tissue so that the barbs penetrate the tissue and emerge from the other side where they are then locked into the retainer piece.

During each of the aforementioned surgical procedures, the tissue is initially gripped or clamped between the cartridge and the anvil such that individual fasteners can be ejected from the cartridge, through the slots, and forced through the clamped tissue. Thereafter, the fasteners are formed by driving them into the depressions formed on the anvil.

A common concern in each of these procedures is hemostasis, or the rate at which bleeding of the target tissue is stopped. It is commonly known that by increasing the amount of pressure applied to a wound, the flow of blood can be limited, thereby decreasing the time necessary to achieve hemostasis. To this end, conventional surgical fastening apparatus generally apply two or more rows of fasteners about the cut-line to compress the surrounding tissue in an effort to stop any bleeding and to join the cut tissue together. Each of the fasteners will generally apply a compressive force to the tissue sufficient to effectuate hemostasis, however, if too much pressure is applied, this can result in a needless reduction in blood flow to the tissue surrounding the cut-line. Accordingly, the joining of tissue together in this manner may result in an elevated level of necrosis, a slower rate of healing, and/or a greater recovery period.

Consequently, it would be advantageous to provide a surgical fastening apparatus capable of limiting the flow of blood in the tissue immediately adjacent the cut tissue to effectuate hemostasis and wound closure, while maximizing blood flow in the surrounding tissue to facilitate healing.

Additionally, when tissue is clamped and compressed between the anvil and cartridge, some of the fluid of the tissue is squeezed out so the tissue is compressed further at the center portions of the cartridge and anvil than at the lateral edges, thereby leaving thicker tissue at the edges. It would therefore be advantageous to provide surgical fasteners which could better accommodate these resulting different tissue thicknesses.

### SUMMARY

The invention provides a surgical fastener applying apparatus according to claim 1.

The present disclosure provides in one aspect a surgical fastener applying apparatus comprising a first jaw containing a first row of first surgical fasteners and a second row of second surgical fasteners and a second jaw containing a first row of first anvil pockets and a second row of second anvil pockets deforming the respective first and second rows of fasteners. At least one of the first fasteners has a first unformed length and forms a single loop configuration having a first formed length to form a first compressive space when deformed by the anvil pocket. At least one of the second fasteners has a second unformed length and forms a single loop configuration having a second formed length to form a second compressive space. The first unformed length is less than the second unformed length and the first formed length is less than the second formed length.

Preferably, the first row of fasteners is positioned inboard of the second row of fasteners and has a plurality of first fasteners defining a compressive space smaller than the compressive space of a plurality of the second fasteners. The apparatus can include in some embodiments a third row of third fasteners positioned outboard (further from the central longitudinal axis) of the second row of fasteners wherein the second formed length is less than a third formed length of at least one of the third fasteners.

Preferably, the fasteners each have a backspan and a pair of legs extending from the backspan, and during formation the legs of the fastener pass each other to form the single loop configuration. In some embodiments, the fastener legs can have a chamfer to create a camming effect to limit interference as the legs pass each other during formation. In some embodiments, in the formed configuration a combined thickness of the legs is about twice a thickness of the backspan.

In some embodiments, the first and second jaws are pivotally attached. In other embodiments, at least one of the jaws is movable along a substantially linear path to move the jaws into approximation.

In some embodiments, the first and second rows of fasteners are arranged in a substantially annular configuration. In other embodiments, the first and second rows of fasteners are arranged in a substantially linear configuration.

In some embodiments depth of at least one of the anvil pockets in the first row is less than a depth of at least one of the anvil pockets in the second row.

The present disclosure also provides a surgical fastener applying apparatus comprising a fastener assembly having a first pair of first rows of fasteners, a second pair of second rows of fasteners and a corresponding first pair of rows of anvil pockets and second pair of rows of anvil pockets to deform the respective fasteners At least one of fasteners of the first pair of rows of fasteners when formed into a single loop configuration applies a first compressive force on tissue and at least one of the fasteners of the second pair of rows of fasteners when formed into a single loop configuration applies a second different compressive force on tissue.

Preferably, the first pair of rows of fasteners is positioned closer to a central longitudinal axis of the fastener assembly and the first compressive force is greater than the second compressive force.

In some embodiments a depth of at least one of the anvil pockets in the first row is less than a depth of at least one of the anvil pockets in the second row.

Preferably, the fasteners each have a backspan and a pair of legs extending from the backspan, wherein a length of the legs of at least one of the first fasteners is less than a length of the legs of at least one of the second fasteners.

The present disclosure also provides a surgical fastener applying cartridge and anvil assembly for use with a surgical fastener applying instrument. The cartridge and anvil assembly comprises a cartridge having a first pair of first rows of fasteners and a second pair of second rows of fasteners and an anvil having first pair of rows of anvil pockets and a second pair of rows of anvil pockets. At least one of the fasteners of the first pair of rows of fasteners when formed into a single loop configuration applies a first compressive force on tissue and at least one of the fasteners of the second pair of rows of fasteners when formed into a single loop configuration applies a second different compressive force on tissue.

Preferably the first pair of rows of fasteners is positioned closer to a central longitudinal axis of the fastener assembly and the first compressive force is greater than the second compressive force. Preferably, the fasteners each have a backspan, and a distance between the fastener backspan and an arc of the legs when formed is less in at least one of the fasteners in the first fastener row than in at least one of the fasteners in the second row.

In some embodiments, a depth of at least one of the anvil pockets in the first row is less than a depth of at least one of the anvil pockets in the second row.

### BRIEF DESCRIPTION OF THE DRAWINGS

Various embodiments of the present disclosure are described herein below with references to the drawings, wherein:
FIG. 1 is a top, perspective view of a surgical fastener applying apparatus having a tool assembly at a distal end thereof for the application of a variable compressive force to tissue by a plurality of surgical fasteners;
FIG. 2 is a partial perspective view of the tool assembly of FIG. 1 with parts separated illustrating a surgical fastener cartridge and an anvil;
FIG. 3 is a side, perspective view of a general representation of one of the plurality of surgical fasteners applied to tissue by the tool assembly of FIG. 1 and including penetrating ends with corresponding chamfers to facilitate the formation of a surgical fastener having a single-loop configuration;
FIG. 4 is a side view of one embodiment of the surgical fastener of FIG. 3 including legs having a first unformed length and shown prior to formation;
FIG. 5A is a cross-sectional view of the surgical fastener of FIG. 4 subsequent to formation and within adjacent tissue segments for the application of a compressive force thereto;
FIG. 5B is side, perspective view of the surgical fastener of FIG. 4 subsequent to formation illustrating the single-loop configuration thereof;
FIG. 6 is a side view of another embodiment of the surgical fastener of FIG. 3 including legs having a second, shorter unformed length and shown prior to formation;
FIG. 7 is a cross-sectional view of the surgical fastener of FIG. 6 subsequent to formation and within adjacent tissue segments for the application of a greater compressive force thereto;
FIG. 8 is a side view of another embodiment of the surgical fastener of FIG. 3 including legs having a third, shorter unformed length and shown prior to formation;
FIG. 9 is a cross-sectional view of the surgical fastener of FIG. 8 subsequent to formation and within adjacent tissue segments for the application of a still greater compressive force thereto;
FIG. 10 is a side, perspective view of a surgical fastener having a standard "B" configuration upon formation;
FIG. 11 is a partial longitudinal, perspective view of the tool assembly of FIG. 2 illustrating a plurality of surgical fasteners arranged into outer, intermediate, and inner rows;
FIG. 12 is a transverse cross-sectional view of the tool assembly of FIG. 2 illustrating the outer, intermediate, and inner rows of surgical fasteners and a plurality of uniformly dimensioned pockets formed in a tissue contacting surface of a first embodiment of the anvil;
FIG. 13 is an enlarged view of the tissue contacting surface of the anvil of FIG. 12 illustrating the plurality of pockets formed therein;
FIG. 14 is a transverse cross-sectional view of the tool assembly of FIG. 2 including an alternate embodiment of the surgical fastener cartridge, which is loaded with a plurality of uniformly dimensioned surgical fasteners, and an alternate embodiment of the anvil, which includes a plurality of pockets having variable dimensions formed in the tissue contacting surface and arranged into outer, intermediate, and inner rows;
FIG. 15 is a side, cross-sectional view of one of the surgical fasteners seen in the cartridge assembly of FIG. 14 shown within adjacent tissue segments and subsequent to formation by one of the pockets comprising the outer rows for the application of a compressive force thereto;
FIG. 16 is a side, cross-sectional view of one of the surgical fasteners seen in the cartridge assembly of FIG. 14 shown within adjacent tissue segments and subsequent to formation by one of the pockets comprising the intermediate rows for the application of a greater compressive force thereto;
FIG. 17 is a side, cross-sectional view of one of the surgical fasteners seen in the cartridge assembly of FIG. 14 shown within adjacent tissue segments and subsequent to formation by one of the pockets comprising the inner rows for the application of a still greater compressive force thereto;
FIG. 18 illustrates an end-to-end anastomosis device for use with an embodiment of the anvil and surgical fastener cartridge of FIG. 2;
FIG. 19 illustrates a surgical fastener applying instrument for use with an embodiment of the anvil and surgical fastener cartridge of FIG. 2;
FIG. 20 is a cross-sectional view of the tool assembly of FIG. 2 including an alternate embodiment of the surgical fastener cartridge;
FIG. 21 illustrates a transverse anastomosis fastener applying instrument for use with the surgical fastener cartridge of FIG. 20;
FIG. 22 is a top, perspective view of an alternate embodiment of the anvil for use with the tool assembly of FIG. 2;
FIG. 23 is an enlarged view of the tissue contacting surface of the anvil of FIG. 22 illustrating a plurality of pockets formed therein of uniform depth and arranged into pairs to facilitate the formation of a surgical fastener having a single-loop configuration;
FIG. 24 is a longitudinal, cross-sectional view of the anvil of FIG. 22 taken through line 24-24 and illustrating the uniform depth of each of the plurality of pockets formed in the tissue contacting surface;
FIG. 25 is a top, perspective view of another alternate embodiment of the anvil;
FIG. 26 is an enlarged view of the tissue contacting surface of the anvil of FIG. 25 illustrating a plurality of pockets formed therein of various depths and arranged into pairs to facilitate the formation of a surgical fastener having a single-loop configuration; and
FIG. 27 is a longitudinal, cross-sectional view of the anvil of FIG. 25 taken through line 27-27 and illustrating the various depths of the plurality of pockets formed in the tissue contacting surface.

### DETAILED DESCRIPTION OF EXEMPLARY EMBODIMENTS

Various exemplary embodiments of the presently disclosed surgical fastener applying apparatus, and method of manufacturing the same, will now be described in detail with reference to the drawings wherein like references numerals identify similar or identical elements. In the drawings and in the description which follows, the term "proximal" will refer to the end the surgical fastener applying apparatus that is closer to the operator during use, while the term "distal" will refer to the end that is further from the operator, as is traditional and conventional in the art. In addition, the term "surgical fastener" should be understood to include any substantially rigid structure formed of a biocompatible material that is suitable for the intended purpose of joining tissue together, including but not being limited to surgical staples, clips, and the like.

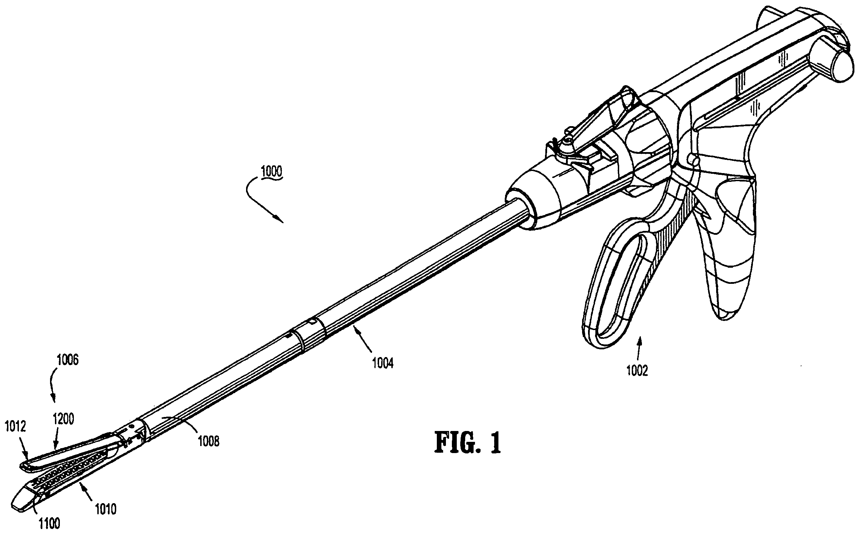

FIG. 1 illustrates an exemplary surgical fastener applying apparatus 1000, of either the re-usable or disposable variety, including a handle 1002, an elongated shaft or endoscopic portion 1004 extending distally therefrom, and a tool assembly 1006 coupled to a distal end 1008 of the elongated shaft 1004. In general, the tool assembly 1006 is adapted to clamp, sequentially fasten together, and sever adjacent tissue segments along a cut-line. During use, the tool assembly of the surgical fastener applying apparatus 1000 is approximated and fired similarly to, and in accordance with other known surgical fastener applying apparatus. For a detailed discussion of the approximation and firing of the surgical fastener applying apparatus 1000, reference is made to commonly assigned U.S. Pat. No. 5,865,361, currently assigned to Tyco Healthcare Group LP.

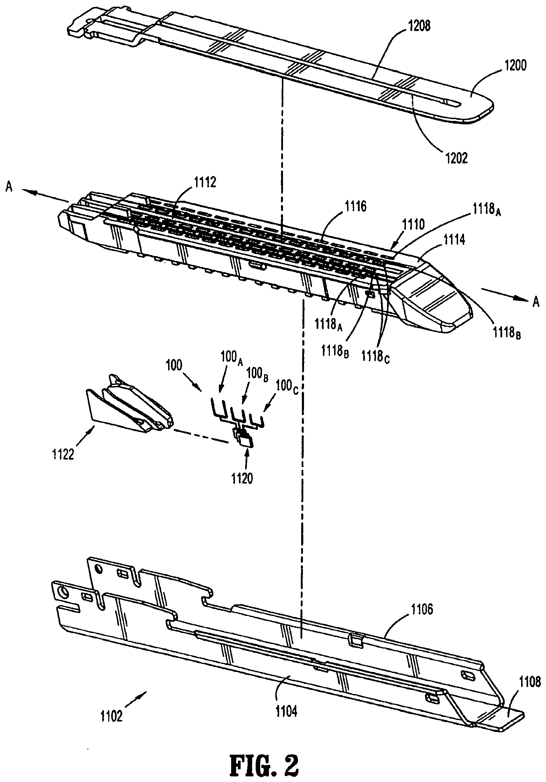

Referring now to FIG. 2 as well, the tool assembly 1006 includes a second jaw 1012 pivotally coupled to a first jaw 1010. In one embodiment of the surgical fastener applying apparatus 1000, the first jaw 1010 of the tool assembly 1006 includes a surgical fastener cartridge 1100 loaded with a plurality of surgical fasteners 100 and the second jaw 1012 includes an anvil 1200 having anvil pockets or depressions for the formation of the surgical fasteners 100. In some embodiments the cartridge and/ or anvil is removable and replaceable.

The surgical fastener cartridge 1100 extends along a longitudinal axis "A-A" and includes a cartridge body 1102 with a pair of side walls 1104, 1106, a bottom wall 1108 and a top wall 1110. The cartridge body 1102 includes a channel or knife slot 1112 that is configured to accommodate longitudinal movement of a knife (not shown) or other cutting element such that tissue may be severed along a cut-line. The top wall 1110 includes a tissue engaging surface 1114, e.g., for maintaining the position of the tissue to be cut, and a plurality of fastener retention slots 1116 arranged into rows, i.e., a pair of outer rows 1118_{A}, a pair of intermediate rows 1118_{B}, and a pair of inner rows 1118_{C}. The pair of outer rows 1118_{A} are disposed laterally outward (outboard) of the intermediate rows 1118_{B}, and are spaced furthest from the knife slot 1112. The pair of intermediate rows 1118_{B} is disposed inboard of the outer rows 1118_{A} and outboard of the inner rows 1118_{C}, thereby positioned between the pair of outer rows 1118_{A} and the pair of inner rows 1118_{C}. The pair of inner rows 1118_{C} is disposed between the pair of intermediate rows 1118_{B} and the knife slot 1112, thereby being closest to the knife slot 1112. Each of the respective outer, intermediate, and inner rows 1118_{A}, 1118_{B}, 1118_{C} are disposed on opposite sides of the knife slot 1112. While the cartridge 1100 is depicted as including three pairs of rows, i.e., the outer, intermediate, and inner rows 1118_{A}, 1118_{B}, 1118_{C}, respectively, fewer or greater number of rows of fastener retention slots 1116 may be included in alternate embodiments of the surgical fastener cartridge 1100.

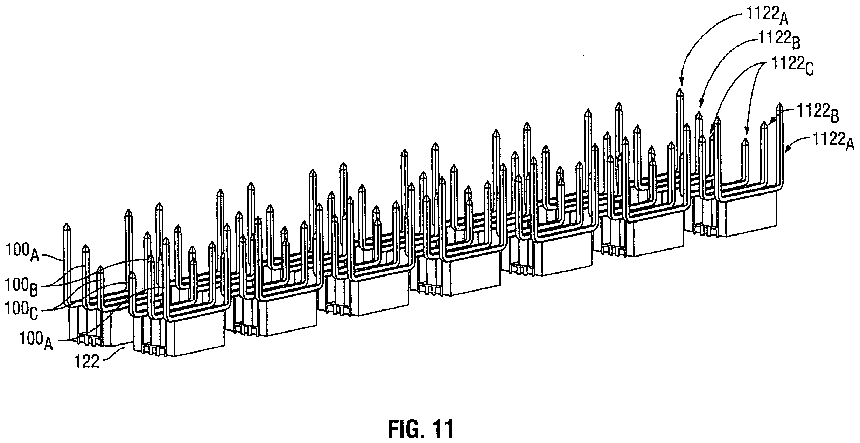

Each fastener retention slot 1116 is configured to receive one of the plurality of surgical fasteners 100 and a correspondingly dimensioned pusher 1120 positioned below. As the pusher 1120, and consequently, the surgical fastener 100 is driven upwardly, i.e., towards the top wall 1110, by a sled 1122. Further details regarding the structure and operation of the tool assembly 1006 and the surgical fastener applying apparatus 1000 can be obtained through reference to U.S. Pat. No. 7,070,083 and U.S. Patent Application Publication No. 20070131732. Plurality of surgical fasteners 100 exit the fastener retention slots 1116, they are deployed in rows, i.e., outer, intermediate, and inner rows 1122_{A}, 1122_{B}, 1122_{C} (FIG. 11), respectively, in the embodiment of the tool assembly 1006 illustrated in FIGS. 1-2. Each of the outer, intermediate, and inner rows 1122_{A}, 1122_{B}, 1122_{C} of surgical fasteners 100 will be deployed and formed within tissue to define corresponding fastener lines disposed on opposite sides of the cut-line created during fastening.

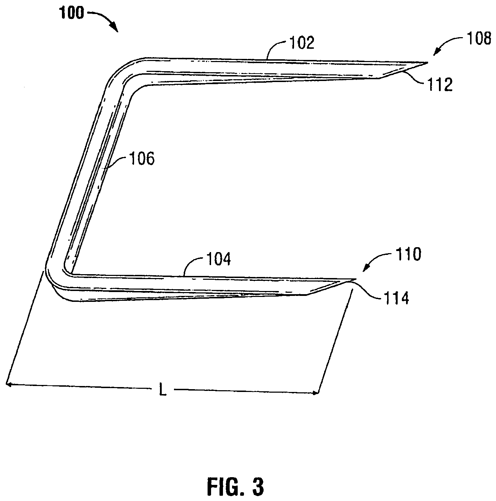

Each surgical fastener 100 as shown in FIG. 3 includes two legs 102, 104 connected by a backspan 106 extending therebetween. The legs 102, 104 extend from the backspan 106 to respective penetrating ends 108, 110, thus defining an unformed length "L". The dimensions of the backspan 106 and the legs 102, 104 can be varied such that the surgical fastener 100 may be used to fasten tissue having varying attributes, such as the thickness thereof or the presence of scar tissue.

The legs 102, 104 and the backspan 106 may define a cross-section having any suitable geometric configuration, including but not being limited to rectangular, oval, square, triangular, trapezoidal, etc. The legs 102, 104 and the backspan 106 may exhibit the same geometrical configuration, or alternatively, the legs 102, 104 and the backspan 106 may exhibit different geometrical configurations, e.g., the legs 102, 104 may exhibit a rectangular cross-section whereas the backspan 106 may exhibit an oval cross-section.

The legs of the fastener may be tapered to facilitate the penetration of tissue as shown, or alternatively, the penetrating ends 108, 110 may not include a taper. In various embodiments, the penetrating ends 108, 110 may define a conical or flat surface, as described in co-pending U.S. Pat. Application Serial No. 11/444,761, filed April 13, 2003. As seen in FIG. 3, the penetrating ends 108, 110 include correspondingly dimensioned chamfers 112, 114 to facilitate the formation of a surgical fastener having a single-loop configuration, as discussed below.

Prior to formation, the legs 102, 104 of each surgical fastener 100 may extend from the backspan 106 such that they are substantially parallel. In the alternative, the legs 102, 104 may converge or diverge from the backspan 106. The present disclosure contemplates that the surgical fasteners 100 may also be directionally biased, as described in U.S. Patent No. 7,398,907.

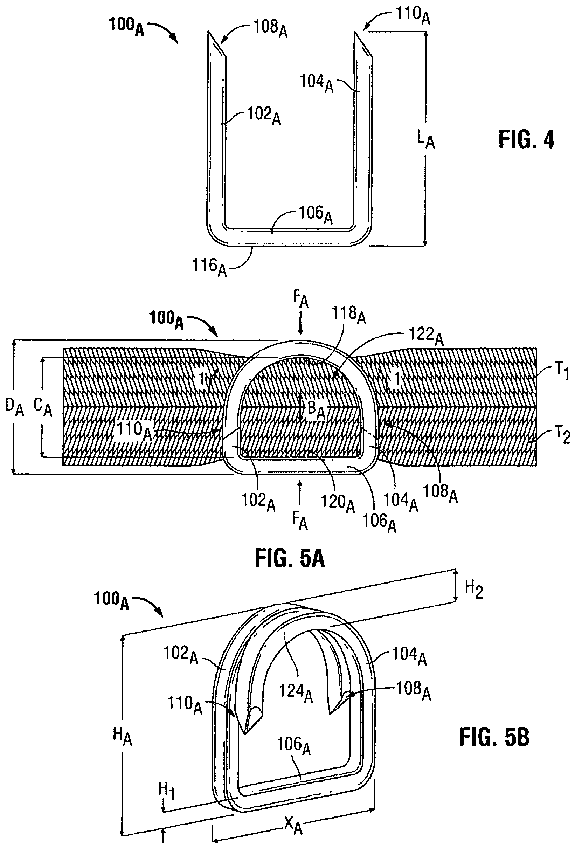

With reference now to FIGS. 4-9, the plurality of surgical fasteners 100 will be discussed prior and subsequent to formation. In the embodiment of the tool assembly 1006 seen in FIGS. 1-2, the plurality of surgical fasteners 100 loaded into the surgical fastener cartridge 1100 includes a first surgical fastener 100_{A}, a second surgical fastener 100_{B}, and a third surgical fastener 100_{C}.

FIGS. 4-5B illustrate the surgical fastener 100_{A} prior to formation and subsequently thereafter. Prior to formation, the legs 102_{A}, 104_{A} define an unformed length "L_{A}" measured from the penetrating ends 108_{A}, 110_{A} to the outer surface 116_{A} of the backspan 106_{A}, thereby defining an unformed length of the fastener. Subsequent to formation of the surgical fastener 100_{A}. the legs 102_{A}, 104_{A} are disposed in crosswise relation such that the surgical fastener 100_{A} defines a single loop. When formed in the single loop configuration, the fastener has a formed length D_{A}. When formed within adjacent tissue segments "T₁", "T₂", the tissue segments "T₁", "T₂" are compressed and maintained in approximation, and compressed between an inner surface 118_{A} of the curved legs 102_{A}, 104_{A} and an inner surface 120_{A} of the backspan 106_{A} within a compressive space 122_{A}. The compression of the tissue segments "T₁", "T₂" creates a biasing force "B_{A}" in the tissue segments "T₁", "T₂" that endeavors to force the legs 102_{A}, 104_{A} outwardly in the direction indicated by arrows 1. The legs 102_{A}, 104_{A} resist yielding, but their length "L_{A}" is such that the legs 102_{A}, 104_{A} are deflected outwardly, albeit a minimal distance, under the influence of the biasing force "B_{A}" to ultimately define a compressive space 122_{A} with a dimension "C_{A}". Maintaining the tissue segments "T₁", "T₂" within the compressive space 122_{A} subjects the tissue segments "T₁", "T₂" to a corresponding compressive force "F_{A}" which limits, but does not completely restrict the flow of blood through the tissue surrounding the surgical fastener 100_{A}. Thus, unnecessary necrosing of the fastened tissue segments "T₁", "T₂" may be prevented or impeded.

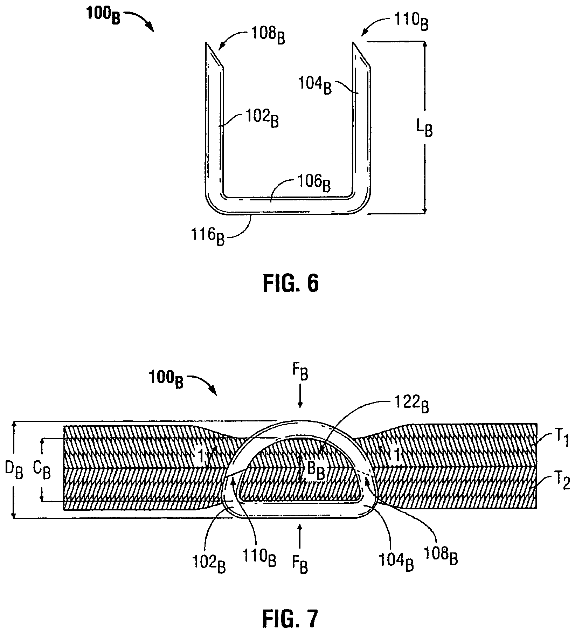

With reference now to FIGS. 6-7, the surgical fastener 100_{B} is shown in its initial and formed conditions, respectively. Prior to formation, the legs 102_{B}, 104_{B} define an unformed length "L_{B}", measured from the penetrating ends 108_{B}, 110_{B} to the outer surface 116_{B} of the backspan 106_{B,} that is less than the length "L_{A}" defined by the legs 102_{A}, 104_{A} of the surgical fastener 100_{A} illustrated in FIGS. 4-5B. Upon formation of the surgical fastener 100_{B}, the legs 102_{B}, 104_{B} are disposed in crosswise relation such that the surgical fastener 100_{B} also defines a single loop. When formed in the single loop configuration, the fastener 100_{B} has a formed length D_{B} which is less than formed length D_{A} of fastener 100_{A}. When the surgical fastener 100_{B} is formed within tissue segments "T₁", "T₂", the compressed tissue segments "T₁", "T₂" exert a biasing force "B_{B}" that endeavors to force the legs 102_{B}, 104_{B} outwardly in the direction indicated by arrows 1. The shorter length "L_{B}" of the legs 102_{B}, 104_{B} allows the legs 102_{B}, 104_{B} to resist yielding to a greater extent than the legs 102_{A}, 104_{A} of the surgical fastener 100_{A} such that a compressive space 122_{B} is ultimately defined with a dimension "C_{B}" that is smaller in comparison to the dimension "C_{A}" of the compressive space 122_{A} illustrated in FIG. 5A. The smaller dimension "C_{B}" of the compressive space 122_{B} results in the application of a corresponding compressive force "F_{B}" to the tissue segments "T₁", "T₂" that is greater than the compressive force "F_{A}" applied by the surgical fastener 100_{A}. Consequently, the flow of blood through the tissue surrounding the surgical fastener 100_{B} is further restricted when compared to the flow of blood through the tissue surrounding the surgical fastener 100_{A}, thereby further facilitating hemostasis. The compressive force "F_{B}" does not completely restrict the flow of blood through the tissue surrounding the surgical fastener 100_{B}, however. Thus, unnecessary necrosing of the fastened tissue segments "T₁", "T₂" may be prevented or impeded.

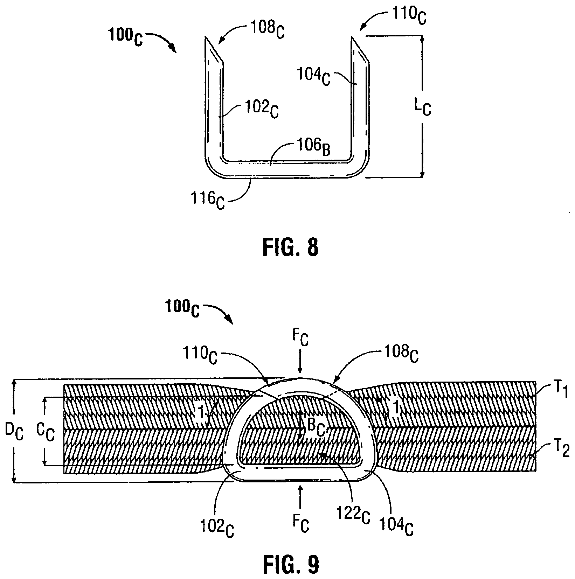

FIGS. 8-9 illustrate the surgical fastener 100_{C} in its initial and formed conditions, respectively. Prior to formation, the legs 102_{C}, 104_{C} define an unformed length "L_{C}" measured from the penetrating ends 108_{C}, 110_{C} to the outer surface 116_{C} of the backspan 106_{C} that is less than the length "L_{B}" defined by the legs 102_{B}, 104_{B} of the surgical fastener 100_{B} illustrated in FIGS. 6-7. Upon formation of the surgical fastener 100_{C}, the legs 102_{C}, 104_{C} are disposed in crosswise relation such that the surgical fastener 100_{C} also defines a single loop. When formed in the single loop configuration, the fastener 100_{C} has a formed length D_{C} which is less than length D_{B} of fastener 100_{B}. When the surgical fastener 100_{C} is formed within tissue segments "T₁", "T₂", the compressed tissue segments "T₁", "T₂" exert a biasing force "B_{C}" that endeavors to force the legs 102_{C}, 104_{C} outwardly in the direction indicated by arrows 1. The shorter length "L_{C}" of the legs 102_{C}, 104_{C} allows the legs 102_{C}, 104_{C} to resist yielding to a greater extent than the legs 102_{B}, 104_{B} of the surgical fastener 100_{B} such that a compressive space 122_{C} is ultimately defined that has a dimension "C_{C}" that is smaller in comparison to the dimension "C_{B}" of the compressive space 122_{B} illustrated in FIG. 7. The smaller dimension "C_{C}" of the compressive space 122_{C} results in the application of a corresponding compressive force "F_{C}" to the tissue segments "T₁", "T₂" that is greater than the compressive force "F_{B}" applied by the surgical fastener 100_{B}. Consequently, the flow of blood through the tissue surrounding the surgical fastener 100_{C} is further restricted when compared to the flow of blood through the tissue surrounding the surgical fastener 100_{B}. The compressive force "F_{B}" applied to the tissue segments "T₁", "T₂" substantially, if not completely restricts the flow of blood through the tissue surrounding the surgical fastener 100_{C}, thereby further facilitating, and effectuating hemostasis.

The length "L_{A}" of the legs 102_{A}, 104_{A}, the length "L_{B}" of the legs 102_{B}, 104_{B}, and the length "L_{C}" of the legs 102_{C}, 104_{C}, as well as the corresponding dimensions "C_{A}", "C_{B}", "C_{C}" of the compressive spaces 122_{A}, 122_{B}, 122_{C} occupied by tissue segments "T₁", "T₂" when the respective surgical fasteners 100_{A}, 100_{B}, 100_{C} are in their formed conditions may be altered or varied in different embodiments to effectuate any desired level of hemostasis and blood flow in the tissue segments "T₁", "T₂".

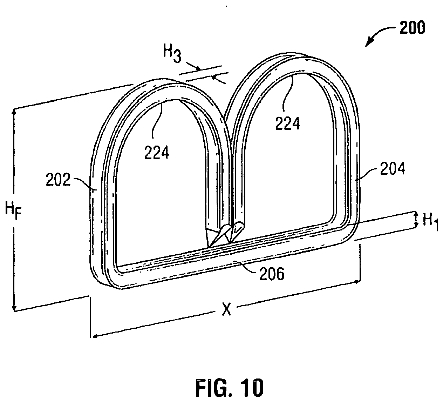

When a surgical fastener is formed to define a single-loop configuration, e.g., the surgical fastener 100_{A} seen in FIGS. 4-5B, for example, the engaging surface 124_{A} of the curved legs 102_{A}, 104_{A} that contacts tissue defines a dimension "H₂" that is twice the thickness "H₁" of the backspan 106_{A} due to the crosswise disposition of the legs 102_{A}, 104_{A}. By contrast, in an otherwise substantially similar surgical fastener 200 exhibiting a standard "B" configuration upon formation such as that shown in Figure 10, the engaging surface 224 of the curved legs 202, 204 that contacts tissue defines a dimension "H₃" that is equal to the thickness "H₁" of the backspan 206, as seen in FIG. 10. As shown in Figure 5B, the tips of the legs 102, 104, when formed point toward the backspan 106 and the arc of the legs 102, 104 extend alongside each other along a substantial portion of their length.

With continued reference to FIGS. 4-5B and 10, to achieve a particular formed height "H_{F}", and thus achieve a particular level of compression in tissue upon fastening, the surgical fastener 200 will generally include a backspan 206 defining a length "X" that is approximately twice the desired formed height "H_{F}". In contrast, to achieve the same formed height, for example formed height "H_{A}", in the single-loop surgical fastener 100_{A}, the backspan 106_{A} need define a length "X_{A}" that is approximately equal to the formed height "H_{A}". Stated differently, to achieve a particular formed height "H_{A}", and thus a particular level of compression within tissue, the employ of a single-loop surgical fastener permits the use of a smaller surgical fastener than the "B" configuration staple.

Smaller surgical fasteners necessarily maintain a smaller amount of tissue between their legs, resulting in a fastener line being more flexible than would otherwise be achievable through the use of larger surgical fasteners. Increased flexibility in the fastener line may result in greater elasticity in the tissue during the recovery period following completion of the surgical procedure, and may result in numerous consequential benefits to the patient during recovery. For example, a more flexible, more elastic fastener line may increase patient mobility while decreasing the likelihood of cat-eyeing about the puncture sites in the tissue created by the legs of the surgical fasteners, thus resulting in increased patient comfort, and perhaps even a shorter recovery period.

Returning now to the figures, and to FIGS. 2 and 11 in particular, the surgical fasteners 100_{A} (FIG. 4), 100_{B} (FIG. 6), 100_{C} (FIG. 8) are shown installed within the surgical fastener cartridge 1100. The surgical fasteners 100_{A} are arranged to define a pair of outer rows 1122_{A}, the surgical fasteners 100_{B} are arranged to define a pair of a pair of intermediate rows 1122_{B}, and the surgical fasteners 100_{C} are arranged to define a pair of a pair of inner rows 1122_{C} that respectively correspond to the outer, intermediate, and inner rows 1118_{A}, 1118_{B}, 1118_{C} of fastener retention slots 1116 formed in the top wall 1110 of the surgical fastener cartridge 1100. Accordingly, the surgical fasteners 100_{A} comprising the pair of outer rows 1122_{A} will be spaced laterally outward, and furthest from the cut-line formed upon fastening, the surgical fasteners 100_{B} comprising the pair of intermediate rows 1122_{B} will be disposed between the surgical fasteners 100_{A} and the fasteners 100_{C} of inner rows 1122_{C}, and the pair of inner rows 1122_{C} will be disposed between the surgical fasteners 100_{B} and cut-line, being closest thereto. Each of the outer, intermediate, and inner rows 1122_{A}, 1122_{B}, 1122_{C} of surgical fasteners will be applied to the tissue such that they define corresponding fastener lines disposed on opposite sides of the cut-line.

The surgical fastener cartridge 1100 seen in FIGS. 1-2 includes outer, intermediate, and inner rows 1122_{A}, 1122_{B}, 1122_{C} exclusively including the surgical fasteners 100_{A}, 100_{B}, 100_{C}, respectively, as best seen in FIG. 11. In other words, each surgical fastener disposed in a particular row will have the same configuration, i.e., legs of the same length. By arranging the surgical fasteners 100_{A}, 100_{B}, 100_{C} in this manner, the surgical fasteners with the shortest leg length resulting in the greatest compressive force, i.e., surgical fasteners 100_{C}, are deployed closest to the cut-line, and the surgical fasteners having longer legs and resulting in lesser compressive forces, i.e., surgical fasteners 100_{B} and 100_{A}, are provided further from the cut-line. Consequently, arranging the surgical fasteners 100_{A}, 100_{B}, 100_{C} in this way minimizes the flow of blood through the tissue immediately adjacent the cut-line and gradually increases the flow of blood through the tissue spaced a greater lateral distance therefrom. It should be appreciated that the length of the legs could be varied to accommodate tissue of different thicknesses and to control tissue compression by the fasteners.

In alternate embodiments, however, the surgical fastener cartridge 1100 may include outer, intermediate, and inner rows 1122_{A}, 1122_{B}, 1122_{C} of different arrangement or comprising a combination of surgical fasteners 100_{A}, 100_{B}, 100_{C} such that a particular row may include a variety of surgical fasteners having different configurations, i.e., legs of different lengths. By providing a variety of surgical fasteners in each row, the flow of blood through the tissue can be controlled longitudinally, along the cut-line, as well laterally as the distance therefrom is increased.

It is also contemplated in some embodiments, some of the surgical fasteners in the cartridge can be the single loop fasteners disclosed herein while other fasteners in the cartridge could be the B-shaped fasteners of Figure 11. These B-shaped fasteners can occupy one of the rows of fasteners or be placed in the rows containing single loop fasteners.

With continued reference to FIG. 11, by loading the surgical fastener cartridge 1100 with a variety of surgical fasteners, and by arranging the surgical fasteners such that those with the shortest legs, i.e., surgical fasteners 100_{C}, are closest to the cut-line and those with the longest legs, i.e., surgical fasteners 100_{A}, are furthest from the cut-line, a greater range of tissue thickness can be effectively fastened, as the thickness of the tissue will generally increase as the distance from the cut-line is also increased. That is, in this manner, the fasteners with the greater distance between the curve of the legs and the backspan are provided on the outer rows where the tissue might be thicker as a result of clamping by the instrument jaws (anvil and cartridge). Accordingly, loading a surgical fastener cartridge with a variety of surgical fasteners having legs of various lengths allows a single surgical fastener cartridge to fasten tissue of varying thickness.

Referring now to FIGS. 1-2 and 12-13, the anvil 1200 and the formation of the plurality of surgical fasteners 100 will be discussed. The anvil 1200 is an elongated member having a tissue contacting surface 1202 with a plurality of pockets 1204 formed therein. The pockets 1204 are arranged into rows, e.g., outer, intermediate, and inner rows 1206_{A}, 1206_{B}, 1206_{C}, respectively. While the anvil 1200 is illustrated as including three pairs of rows, i.e., outer, intermediate, and inner rows 1206_{A}, 1206_{B}, 1206_{C}, respectively, fewer and greater numbers of rows of pockets 1204 may be included in alternate embodiments of the anvil 1200 to correspond to the rows of fasteners. The pockets 1204 are arranged such that the respective outer, intermediate, and inner rows 1206_{A}, 1206_{B}, 1206_{C} correspond in location to the outer, intermediate, and inner rows 1118_{A}, 1118_{B}, 1118_{C} of fastener retention slots 1116 (FIG. 2) formed in the top wall 1110 of the cartridge body 1102, such that each pocket 1204 is substantially aligned with a corresponding fastener retention slot 1116 during firing of the surgical fastener applying apparatus 1000. Like the surgical fastener cartridge 1100, the anvil 1200 includes a channel or slot 1208 that is configured to accommodate movement of a knife, or other such cutting element (not shown).

The pockets 1204 are configured and dimensioned to deform the plurality of surgical fasteners 100, e.g., the surgical fasteners 100_{A}, 100_{B}, 100_{C}, so as to achieve the single-loop formed configurations discussed above (*see* FIGS. 5A-5B, 7, 9). More particularly, each of the pockets 1204 defines an arcuate recess 1210 having a forming surface 1212 that extends away from the tissue contacting surface 1202. The pockets 1204 have substantially equal depths "D" and substantially equal widths "W" that are dimensioned to accommodate both of the legs 102, 104 (FIG. 3) of the surgical fasteners 100 in crosswise relation during formation.

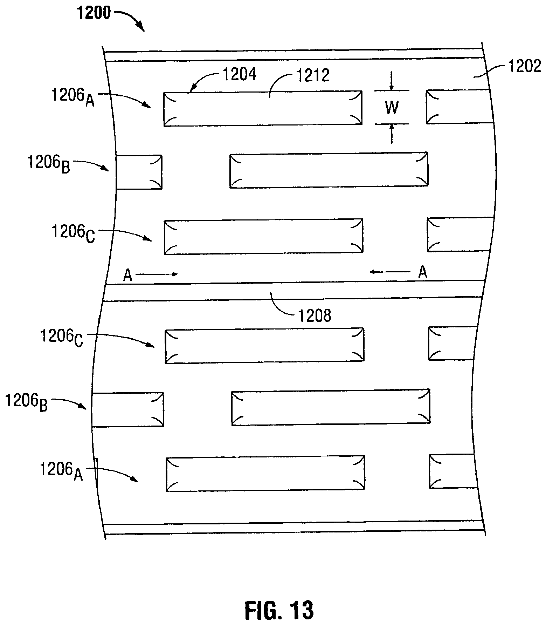

Upon firing the surgical fastener applying apparatus 1000 (FIG. 1), each of the plurality of surgical fasteners 100 is discharged from the surgical fastener cartridge 1100 through a fastener retention slot 1116 such that the respective penetrating ends 108, 110 of each of the legs 102, 104 are forced into engagement with the forming surface 1212 of a corresponding pocket 1204 formed in the anvil 1200. Thereafter, as each surgical fastener 100 is continually advanced towards the anvil 1200, the forming surface 1212 of each pocket 1204 guides the legs 102, 104 such that they are deformed inwardly in the direction of arrows "A" (Figure 13). Should the penetrating ends 108, 110 come into contact during formation, the chamfers 112, 114 (Figure 3) create a camming effect which allows the legs 102, 104 to pass each other without substantial interference. Upon realizing their formed configuration, the legs 102, 104 are disposed in crosswise relation such that the surgical fastener 100 achieves the single-loop configuration discussed above in connection with the discussion of the surgical fasteners 100_{A}, 100_{B}, 100_{C} respectively illustrated in FIGS. 5A-5B, 7, and 9.

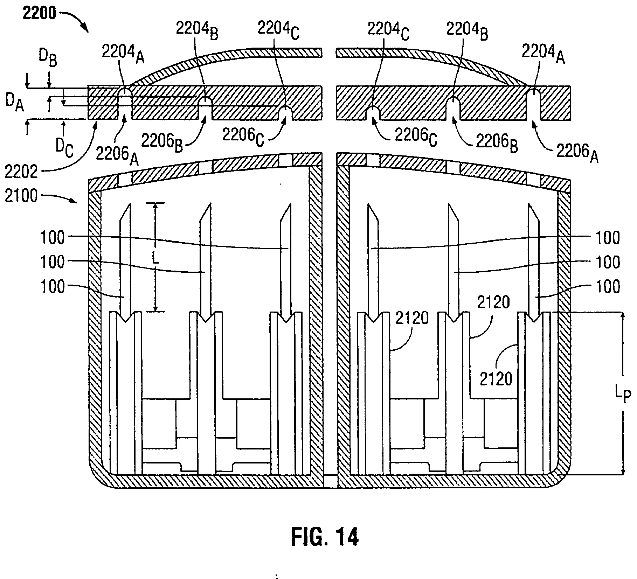

FIG. 14 illustrates an alternate embodiment of the surgical fastener cartridge and the anvil for use with the tool assembly 1006 (FIG. 1), referred to generally as surgical fastener cartridge 2100 and anvil 2200.

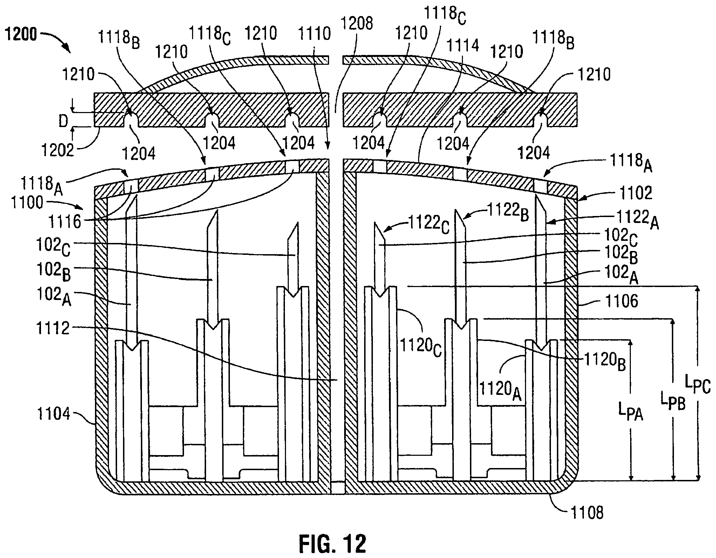

The surgical fastener cartridge 2100 is loaded with a plurality of surgical fasteners 100 (FIG. 3) each having legs 102, 104 that define the same unformed length "L". Consequently, the surgical fastener cartridge 2100 also includes pushers 2120 that define equal heights "L_{P}", in contrast to the pushers 1120_{A}, 1120_{B}, 1120_{C} of the surgical faster cartridge 1100 defining differing heights "L_{PA}", "L_{PB}", "L_{PC}" (L_{PA} < L_{PB} < L_{PC}) corresponding to the varying surgical fasteners 100_{A}, 100_{B}, 100_{C} engaged respectively thereby upon firing (*see* FIG. 12).

The anvil 2200 includes a plurality of pockets 2204_{A}, 2204_{B}, 2204c that define respective depths "D_{A}", "D_{B}", "D_{C}", measured from the tissue contacting surface 2202, that decrease inwardly from an outer row 2206_{A} to an intermediate row 2206_{B} to an inner row 2206_{C}. Accordingly, the pockets 2204_{A} comprising the outer rows 2206_{A} are deeper than the pockets 2204_{B} comprising the intermediate rows 2206_{B}; and the pockets 2204_{B} comprising the intermediate rows 2206_{B} are deeper than the pockets 2204_{C} comprising the inner rows 2206_{C}, i.e. D_{A} > D_{B}> D_{C}.

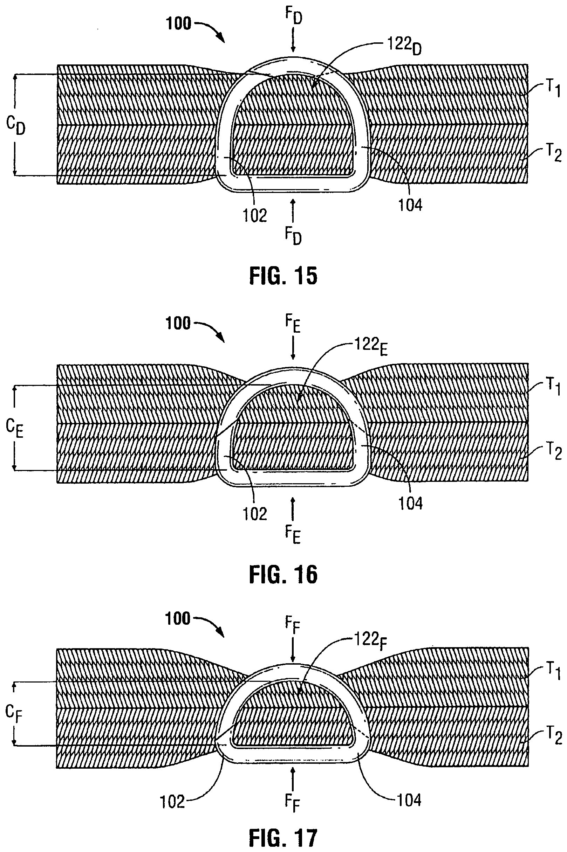

Referring now to FIG. 15 as well, one of the plurality of surgical fasteners 100 is illustrated within adjacent tissue segments "T₁", "T₂" subsequent to the formation thereof through engagement with the pockets 2204_{A} comprising the outer rows 2206_{A} formed in the anvil 2200. The depth "D_{A}" of the pockets 2204_{A} comprising the outer rows 2206_{A} facilitates the formation of a compressive space 122_{D} which maintains the tissue segments "T₁", "T₂" in approximated relation. The compressive space 122_{D} results in the application of a corresponding compressive force "F_{D}" to the tissue segments "T₁", "T₂", as previously discussed, for example, with respect to FIGS. 5A. The compressive force "F_{D}" restricts the flow of blood through the tissue surrounding the surgical fastener 100, thereby facilitating hemostasis. However, the depth "D_{A}" of the pockets 2204_{A} comprising the outer rows 2206_{A} and the dimension "C_{D}" of the compressive space 122_{D} are such that the resulting compressive force "F_{D}" does not completely restrict the flow of blood through the tissue. Thus, unnecessary necrosing of the fastened tissue may be prevented or impeded.

Referring now to FIG. 16, a surgical fastener 100 is illustrated within adjacent tissue segments "T₁", "T₂" subsequent to the formation thereof through engagement with the pockets 2204_{B} comprising the intermediate rows 2206_{B} formed in the anvil 2200 (FIG. 14). The shallower depth "D_{B}" of the pockets 2204_{B} comprising the intermediate rows 2206_{B} results in greater deformation of the legs 102, 104 of the surgical fastener 100 when compared to the deformation resulting from the engagement of the legs 102, 104 with the pockets 2204_{A} comprising the outer rows 2206_{A} (*see* FIG. 15) such that a compressive space 122_{E} having a dimension "C_{E}" is defined. The formed length of the fastener of FIG. 16 is less than the formed length of the fastener of FIG. 15. Accordingly, the dimension "C_{E}" of the compressive space 122_{E} is less than the dimension "C_{D}" of the compressive space 122_{D} seen in FIG. 15, thereby resulting in the application of a corresponding compressive force "F_{E}" that is greater than the compressive force "F_{D}". Consequently, the flow of blood through the tissue surrounding the surgical fastener 100 formed through engagement with the pockets 2204_{B} comprising the intermediate rows 2206_{B} formed in the anvil 2200 is less than the flow of blood through the tissue surrounding the surgical fastener 100 formed through engagement with the pockets 2204_{A} comprising the outer rows 2206_{A}, thereby further facilitating hemostasis. However, the depth "D_{B}" of the pockets 2204_{B} comprising the intermediate rows 2206_{B} and the dimension "C_{E}" of the compressive space 122_{E} are such that the resulting compressive force "F_{E}" does not completely restrict the flow of blood through the tissue. Thus, unnecessary necrosing of the fastened tissue may be prevented or impeded.

FIG. 17 illustrates a surgical fastener 100 within adjacent tissue segments "T₁", "T₂" subsequent to the formation thereof through engagement with the pockets 2204_{C} comprising the inner rows 2206_{C} formed in the anvil 2200. The shallower depth "D_{C}" of the pockets 2204_{C} comprising the inner rows 2206_{C} results in greater deformation of the legs 102, 104 when compared to the deformation resulting from the engagement of the legs 102, 104 with the pockets 2204_{B} comprising the intermediate rows 2206_{B} (*see* FIG. 16) such that a compressive space 122_{F} having a dimension "C_{F}" is defined. The formed length of the fastener of FIG. 17 is less than the formed length of the fastener of FIG. 16. Accordingly, the dimension "C_{F}" of the compressive space 122_{F} is less than the dimension "C_{E}" of the compressive space 122_{E} seen in FIG. 16, thereby resulting in the application of a corresponding compressive force "F_{F}" that is greater than the compressive force "F_{E}". Consequently, the flow of blood through the tissue surrounding the surgical fastener 100 formed through engagement with the pockets 2204_{C} comprising the inner rows 2206_{C} formed in the anvil 2200 is less than the flow of blood through the tissue surrounding the surgical fastener 100 formed through engagement with the pockets 2204_{B} comprising the intermediate rows 2206_{B}. The depth "D_{C}" of the pockets 2204_{C} comprising the inner rows 2206_{C} and the dimension "C_{F}" of the compressive space 122_{F} are such that the resulting compressive force "F_{F}" substantially, if not completely restricts the flow of blood through the tissue, thereby further facilitating, and effectuating hemostasis.

In the particular embodiment illustrated in FIG. 14, the anvil 2200 includes outer, intermediate, and inner rows 2206_{A}, 2206_{B}, 2206_{C} which exclusively comprise pockets 2204 of a particular depth. Stated differently, in the embodiment of FIG. 14, each of the pockets 2204_{A} comprising the outer rows 2206_{A} has a substantially identical depth "D_{A}", each of the pockets 2204_{B} comprising the intermediate rows 2206_{B} has a substantially identical depth "D_{B}", and each of the pockets 2204_{C} comprising the inner rows 2206_{C} has a substantially identical depth "D_{C}". Accordingly, the flow of blood through the tissue surrounding the surgical fasteners 100 formed through engagement with the pockets 2204_{A} comprising the outer rows 2206_{A} is less restricted when compared to the flow of blood through the tissue surrounding the surgical fasteners 100 formed through engagement with the pockets 2204_{B} comprising the intermediate rows 2206_{B}, which is less restricted than the flow of blood through the tissue surrounding the surgical fasteners 100 formed through engagement with the pockets 2204_{C} comprising the inner rows 2206_{C}. Consequently, the surgical fasteners 100 formed through engagement with the pockets 2204 formed in the anvil 2200 will minimize blood flow through the tissue immediately adjacent the cut-line upon formation and permit increased blood flow through the tissue spaced a greater distance from the cut-line.

In alternate embodiments, the present disclosure contemplates outer rows 2206_{A}, intermediate rows 2206_{B}, and inner rows 2206_{C} which include pockets 2204 of different arrangements of the rows and/or various depths. That is, one or more of the rows may include one or more pockets 2204_{A} having a depth "D_{A}", one or more pockets 2204_{B} having a depth "D_{B}" and/or one or more pockets 2204_{C} having a depth "D_{C}" spaced longitudinally along the anvil 2200. The inclusion of pockets 2204 having variable depths in each row 2206 will control blood flow through the tissue longitudinally, i.e., along the cut-line, as well laterally as the distance therefrom is increased.

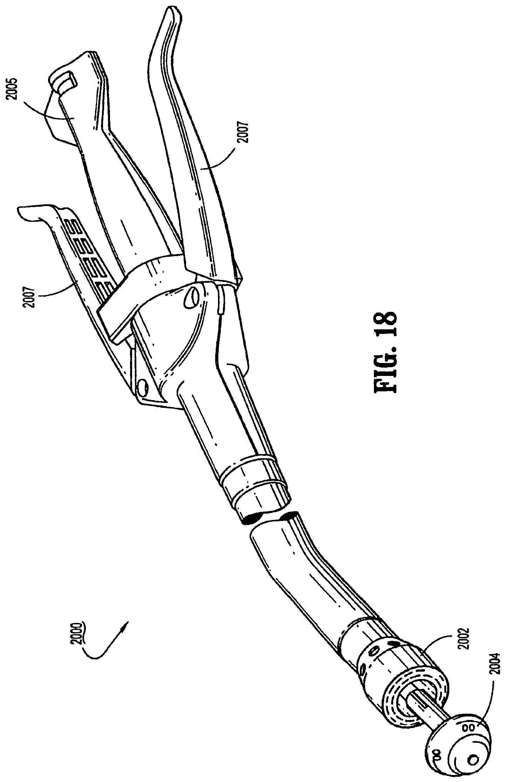

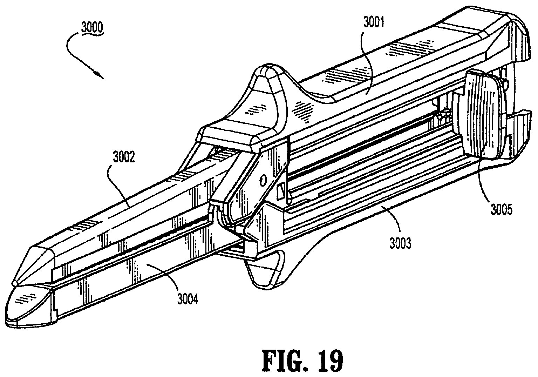

While the surgical fastener cartridges 1100, 2100 and the anvils 1200, 2200 discussed above have been described in connection with the surgical fastener applying apparatus 1000 illustrated in FIG. 1 adapted for use in laparoscopic procedures, the surgical fastener cartridges 1100, 2100 and the anvils 1200, 2200 may be adapted for use with any surgical instrument suitable for the intended purpose of applying a plurality of surgical fasteners to a section of tissue and severing the tissue along a cut-line. For example, the surgical fastener cartridges 1100, 2100 and the anvils 1200, 2200 may be adapted for use with an end-to-end anastomosis device 2000, as seen in FIG. 18, a surgical fastening instrument 3000 for use during a gastro-intestinal anastomotic fastening procedure, as seen in FIG. 19, or, for example, any of the surgical fastener applying apparatus discussed in U.S. Pat. Nos. 6,045,560; 5,964,394; 5,894,979; 5,878,937; 5,915,616; 5,836,503; 5,865,361; 5,862,972; 5,817,109; 5,797,538; and 5,782,396. In the apparatus of Figure 18, a plurality of fasteners are arranged in substantially annular rows within the cartridge or fastener supporting portion 2002. Anvil pockets are formed in anvil portion 2004. Approximation of the cartridge and anvil, e.g. retraction of the anvil 2004 by rotation of approximation knob (wing nut) 2005 clamps tissue between the anvil 2004 and cartridge 2002. Squeezing of handles 2007 advances the fasteners through the tissue and into contact with the anvil pockets where they are formed into the single loop configuration, providing varying compressive forces on the tissue due to the varying length of the fastener legs, varying depths of the anvil pockets or varying of both. A knife is advanced with firing of the instrument.

In the surgical fastener applying apparatus 3000 of Figure 19, a plurality of surgical fasteners are applied into either side of a target section of tissue (not explicitly shown). A knife is advanced with the firing of the fasteners. The fasteners are supported within the cartridge or fastener supporting portion 3002 and the anvil pockets are formed in the anvil supporting portion 3004. The instrument halves 3001 and 3003 are clamped together to approximate the cartridge and anvil, and movement of firing knob 3005 sequentially fires the fasteners through the tissue and into contact with the anvil pockets where they are formed into the single loop configuration, providing varying compressive forces on the tissue due to the varying length of the fastener legs, varying depths of the anvil pockets or varying of both.

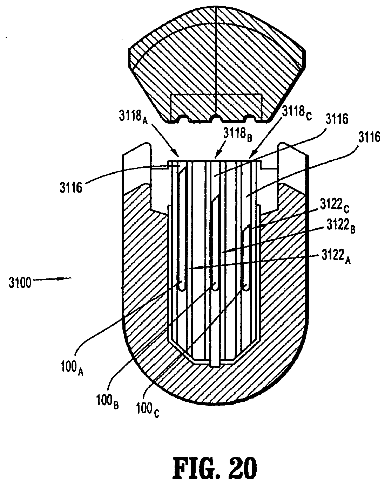

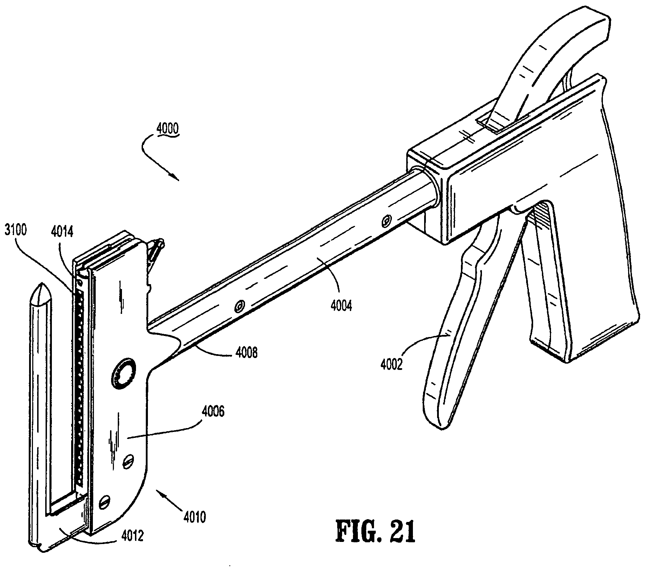

FIG. 20 illustrates an alternate embodiment of the surgical fastener cartridge, referred to generally as surgical fastener cartridge 3100, that is adapted for use with the surgical fastener applying apparatus 4000 seen in FIG. 21

The surgical fastener applying apparatus 4000 includes a handle 4002, an elongated portion 4004 extending distally from the handle 4002, and a frame 4006 extending from a distal end 4008 of the elongated portion 4004. The surgical fastener applying apparatus 4000 further includes an anvil member 4012. A surgical fastener cartridge receiving portion 4014 of the frame receives cartridge 3100. The surgical fastener applying apparatus 4000 may be of either the re-usable or disposable variety.

The surgical fastener cartridge 3100 is similar to the surgical fastener cartridges discussed above in that it contains multiple rows of fasteners. While the surgical fastener cartridge 3100 is depicted as including surgical fasteners having legs of different lengths, i.e., surgical fasteners 100_{A} (*see also* FIG. 4), 100_{B} (*see also* FIG. 6), 100_{C} (*see also* FIG. 8), the surgical fastener cartridge 3100 may alternatively be loaded with a plurality of surgical fasteners having legs with substantially equal lengths cooperating with an anvil having rows of differing anvil pocket depths as discussed above with respect to Figure 14.

It is also contemplated that as in the other embodiments discussed herein, alternatively a cartridge with surgical fasteners having legs of different lengths can be used with an anvil of different pocket depths as long as the rows of staples are formed and placed to perform the functions described herein.

The surgical fastener cartridge 3100 illustrated in Figure 20 includes a plurality of fastener retention slots 3116 that are arranged into a single outer row 3118_{A}, a single intermediate row 3118_{B}, and a single inner row 3118_{C}, respectively, in contrast to the pairs of rows previously discussed with respect to the surgical fasteners cartridges 1100, 2100. Accordingly, the surgical fasteners 100_{A}, 100_{B}, 100_{C} are also arranged into a single outer row 3122_{A}, a single intermediate row 3122_{B}, and a single inner row 3122_{C}, respectively. The terms "outer", "intermediate", and "inner" are relative terms which refer to the placement of the surgical fasteners 100_{A}, 100_{B}, 100_{C} in relation to the cut-line formed in the tissue subsequent to fastening. While the surgical fastener cartridge 3100 is shown as including three rows of retention slots, i.e., the outer row 3118_{A}, the intermediate row 3118_{B}, and the inner row 3118_{C}, and three corresponding rows of surgical fasteners, i.e., the outer row 3122_{A}, the intermediate row 3122_{B}, and the inner row 3122_{C}, alternate embodiments of the surgical fastener 3100 may include fewer or greater numbers (e.g. pairs) of rows of fastener retention slots 3116 and surgical fasteners (and corresponding anvil pockets).

During use, after approximation of the cartridge receiving portion 4014 and anvil portion 4012, the surgical fastener applying apparatus 4000 simultaneously applies the plurality of surgical fasteners 100_{A}, 100_{B}, 100_{C} to tissue to define individual fastener lines, i.e., an outer fastener line, an intermediate fastener line, and an inner fastener line in the embodiment illustrated in FIGS. 20-21. As an example, the surgical fastener applying apparatus 4000 may be the transverse anastomosis fastening instrument disclosed in U.S. Pat. No. 7,070,083 and 5,964,394. However, any surgical fastener applying apparatus suitable for the intended purpose of fastening tissue in the aforedescribed manner may be employed. Subsequent to the fastening of tissue, a scalpel, or the like, may be used to create a cut-line in the tissue adjacent the surgical fasteners 100_{C} comprising the inner row 3122_{C}. Alternatively, however, the surgical fastener cartridge 3100 may include a channel configured to accommodate a knife, or other such cutting element, as discussed above with respect to the surgical fastener cartridges 1100 (*see* FIGS. 2, 12) and 2100 (FIG. 14) and described in U.S. Patent Application Publication No. 20070131732.

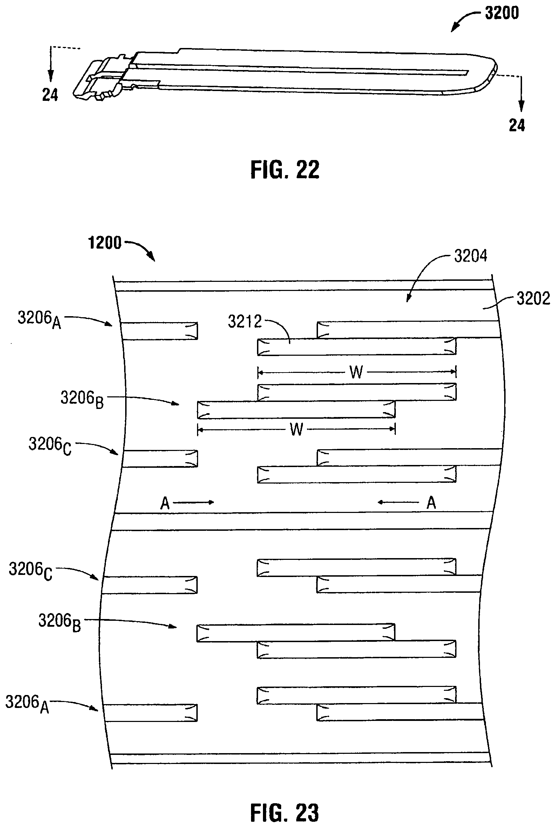

Referring now to FIGS. 22-24, another embodiment of the anvil is disclosed, referred to generally as anvil 3200. In particular, the anvil 3200 is adapted for use with the surgical fastener cartridge 1100 seen in FIG. 2, i.e., a surgical fastener cartridge including outer, intermediate, and inner rows 1118_{A}, 1118_{B}, 1118_{C} comprised of surgical fasteners 100_{A}-100_{C} respectively including legs 102_{A}-102_{C}, 104_{A}-104_{C} defining variable lengths "L_{A}"-"L_{C}" (*see* FIGS. 4, 6, 8, 11). While the surgical fastener cartridge 1100 is illustrated in connection with surgical fasteners having legs with chamfered penetrating ends, i.e., surgical fasteners 100_{A}-100_{C}, through reference to the following description of the anvil 3200, it should be appreciated that the anvil 3200 may be used to form surgical fasteners including legs with either chamfered or standard penetrating ends.

The anvil 3200 will be discussed with respect to its differences from anvil 1200. As an illustrative example, the anvil 3200 will be discussed with respect to the formation of a surgical fastener 100_{A} included in the outer row 1118_{A} of the surgical fastener cartridge 1100, as seen in FIG. 11.

The plurality of pockets 3204 formed in the tissue contacting surface 3202 are provided in pairs and are arranged into outer, intermediate, and inner rows 3206_{A}, 3206_{B}, 3206_{C}, respectively. The arrangement of the pockets 3204 into fewer and greater numbers of rows in alternate embodiments is also within the scope of the present disclosure.

The pockets 3204 in each pair are offset from each other and extend in collinear relation to substantially proscribe interference of the legs 102_{A}, 104_{A} during formation by directing the legs 102_{A}, 104_{A} past each other. The pockets 3204 are each configured to deform the legs 102_{A}, 104_{A} of the surgical fastener 100_{A} upon engagement therewith to achieve the formed single loop configuration seen in FIG. 5B. More particularly, each pocket 3204 defines a width "W" that is dimensioned to receive one of the legs 102_{A}, 104_{A} of the surgical fastener 100_{A}, and each pocket 3204 is configured as an arcuate recess 3210 having a forming surface 3212 that extends away from the tissue contacting surface 3202 to define a substantially equal depth "D". As the surgical fastener 100_{A} is continually advanced towards the anvil 3200, the forming surfaces 3212 guide the legs 102_{A}, 104_{A} such that they are deformed inwardly in the direction of arrows "A". The offset relationship of the pockets 3204 in each pair results in a surgical fastener 100_{A} having legs 102_{A}, 104_{A} that pass each other during formation and are disposed in crosswise relation such that the surgical fastener 100_{A} achieves the single-loop configuration seen in FIG. 5B upon formation.

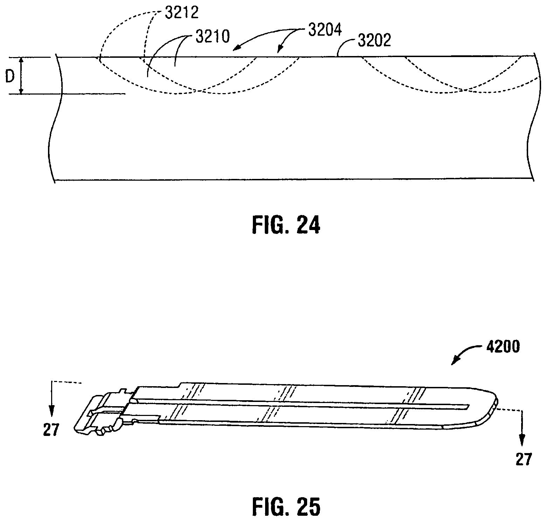

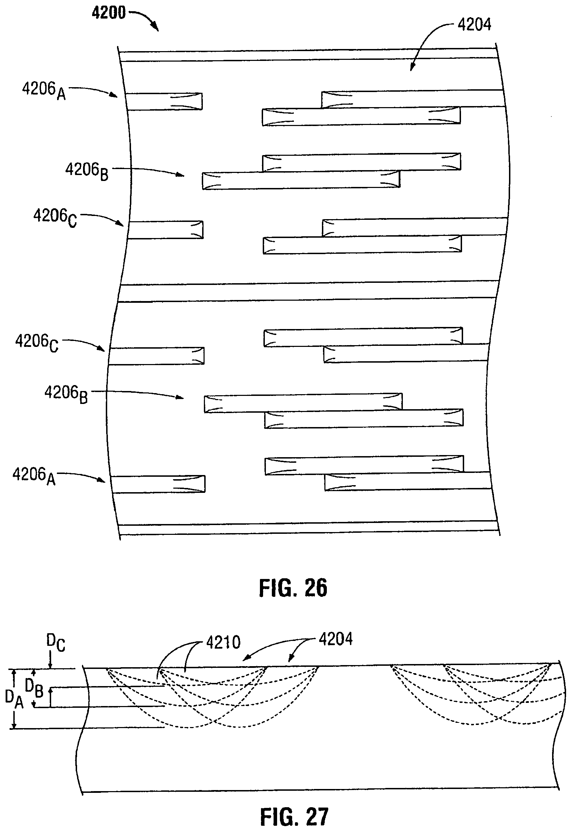

Referring now to FIGS. 25-27, another embodiment of the anvil is disclosed, referred to generally as anvil 4200. In particular, the anvil 4200 is adapted for use with the surgical fastener cartridge 2100 seen in FIG. 14, i.e., a surgical fastener cartridge including outer, intermediate, and inner rows of surgical fasteners having legs defining substantially equal lengths. The anvil 4200 is substantially identical to the anvil 3200 discussed above and illustrated in FIGS. 22-24, but for the configuration of the pockets 4204, and accordingly, the anvil 4200 will only be discussed with respect thereto.

In contrast to the anvil 3200, the pockets 4204 formed in the anvil 4200 are configured as arcuate recesses 4210 defining depths "D_{A}", "D_{B}", "D_{C}" that decrease inwardly from outer rows 4206_{A} to intermediate rows 4206_{B} to inner rows 4206_{C}. Accordingly, the pockets 4204 comprising the outer rows 4206_{A} are deeper than the pockets 4204 comprising the intermediate rows 4206_{B}, and the pockets 4204 comprising the intermediate rows 4206_{B} are deeper that the pockets 4204 comprising the inner rows 4206_{C}, i.e., "D_{A}"> "D_{B}"> "D_{C}".

The varying depths "D_{A}", "D_{B}", "D_{C}" of the pockets 4204 respectively comprising the outer, intermediate, and inner rows 4206_{A}, 4206_{B}, 4206_{C} facilitate the formation of surgical fasteners 100 in the manner discussed above with respect to the anvil 2200 seen in FIG. 14. Accordingly, a surgical fastener 100 formed through engagement with a pocket 4204 included in the outer rows 4206_{A} will exhibit the single-loop configuration and corresponding dimensions illustrated in FIG. 15, a surgical fastener 100 formed through engagement with a pocket 4204 included in the intermediate rows 4206_{B} will exhibit the single-loop configuration and corresponding dimensions illustrated in FIG. 16, and a surgical fastener 100 formed through engagement with a pocket 4204 included in the inner rows 4206_{C} will exhibit the single-loop configuration and corresponding dimensions illustrated in FIG. 17.

While the anvils 3200, 4200 have been described and characterized for use with the tool assembly 1006 (FIGS. 1-2) and the surgical fastener applying apparatus 1000 (FIG. 1), it should be appreciated that the anvils 3200, 4200 may be adapted for use with any of the surgical fastener applying apparatus discussed herein, e.g., the end-to-end anastomosis device 2000 illustrated in FIG. 18, the surgical fastening instrument 3000 illustrated in FIG. 19, and the surgical fastener applying apparatus 4000 illustrated in FIG. 21.

The present disclosure is not limited to the precise embodiments discussed herein above, and various other changes and modifications may be contemplated by one skilled in the art without departing from the scope of the present disclosure. For example, the surgical fasteners described herein above may be formed from a variety of surgically acceptable materials including titanium, plastics, bio-absorbable materials, etc. Although the illustrative embodiments of the present disclosure have been described herein with reference to the accompanying drawings, the above description, disclosure, and figures should not be construed as limiting, but merely as exemplary of various embodiments.

## Claims

1. A surgical fastener applying apparatus (1000) comprising a first jaw (1010) and a second jaw (1012), the first jaw containing a first row (1122c) of first surgical fasteners (100c) and a second row (1122b) of second surgical fasteners (100b), the second jaw containing a first row (1206c, 2206c) of first anvil pockets (1204, 2204) and a second row (1206b, 2206b) of second anvil pockets, the first and second rows of anvil pockets deforming the respective first and second rows of fasteners, at least one of the first fasteners having a first unformed length (Lc) and forming a configuration having a first formed length (Dc) to form a first compressive space (122c) when deformed by the anvil pocket, and at least one of the second fasteners having a second unformed length (Lb) and forming a configuration having a second formed length (Db) to form a second compressive space (122b) deformed by the anvil pocket, wherein the first unformed length is less than the second unformed length and the first formed length is less than the second formed length; and wherein each of the at least one of the first and second fasteners has a backspan (106) and a pair of legs (102, 104) extending from the backspan; **characterised in that** during formation the legs of each pair of legs, pass each other to form a single loop configuration in which the legs are disposed in crosswise relation and the at least one of the first and second fasteners have the first and second formed length.

2. The fastener applying apparatus of claim 1, wherein the first row of fasteners is positioned inboard of the second row of fasteners and each of a plurality of the first fasteners defines a smaller compressive space than the compressive space defined by each of a plurality of the second fasteners.

3. The fastener applying apparatus of claims 1 or 2, the first jaw further comprising a third row (1122a) of third fasteners (100a), at least one of the third fasteners having a third formed length (Da) when formed in a single loop configuration, the third row of fasteners being positioned outboard of the second row of fasteners, the second formed length being less than the third formed length.

4. The fastener applying apparatus of any preceding claim, wherein each of the fastener legs has a chamfer (112, 114) to create a camming effect to limit interference as the legs pass each other during formation.

5. The fastener applying apparatus of any preceding claim, wherein in the formed configuration a combined thickness of the legs is about twice a thickness of the backspan.

6. The fastener applying apparatus of claim 1, wherein the first and second jaws are pivotally attached or wherein at least one of the first and second jaws is movable along a substantially linear path to move the anvil jaws into approximation.

7. The fastener applying apparatus of any preceding claim, wherein the first and second rows of fasteners are arranged in a substantially annular configuration or wherein the first and second rows of fasteners are arranged in a substantially linear configuration.

8. The fastener applying apparatus of any preceding claim, wherein a depth of at least one of the anvil pockets in the first row is less than a depth of at least one of the anvil pockets in the second row.

9. The fastener applying apparatus of any preceding claim, wherein at least one of the fasteners of the first pair of rows of fasteners when formed into a single loop configuration applies a first compressive force on tissue and at least one of the fasteners of the second pair of rows of fasteners when formed into a single loop configuration applies a second different compressive force on tissue.

## Patentansprüche

1. Chirurgische Befestigungselement-Anbringungsvorrichtung (1000), die eine erste Backe (1010) und eine zweite Backe (1012) umfasst, wobei die erste Backe eine erste Reihe (1122c) von ersten chirurgischen Befestigungselementen (100c) und eine zweite Reihe (1122b) von zweiten chirurgischen Befestigungselementen (100b) enthält, die zweite Backe eine erste Reihe (1206c, 2206c) von ersten Ambosstaschen (1204, 2204) und eine zweite Reihe (1206b, 2206b) von zweiten Ambosstaschen enthält, die ersten und zweiten Reihen von Ambosstaschen die entsprechenden ersten und zweiten Reihen von Befestigungselementen verformen, wobei wenigstens eines der ersten Befestigungselemente eine erste ungeformte Länge (Lc) hat und eine Konfiguration mit einer ersten geformten Länge (Dc) formt, um einen ersten kompressiven Raum (122c) zu formen, wenn es durch die Ambosstasche verformt wird, und wenigstens eines der zweiten Befestigungselemente eine zweite ungeformte Länge (Lb) hat und eine Konfiguration mit einer zweiten geformten Länge (Db) formt, um einen zweiten kompressiven Raum (122b) zu formen, verformt durch die Ambosstasche, wobei die erste ungeformte Länge geringer als die zweite ungeformte Länge ist und die erste geformte Länge geringer als die zweite geformte Länge ist und wobei jedes von dem wenigstens einen der ersten und zweiten Befestigungselemente einen Rückensteg (106) und ein Paar von Beinen (102, 104), die sich von dem Rückensteg erstrecken, hat;
**dadurch gekennzeichnet, dass** die Beine jedes Paars von Beinen während einer Verformung einander passieren, um eine Einzelschlaufenkonfiguration zu formen, in der die Beine in einer überkreuzten Beziehung angeordnet sind und das wenigstens eine der ersten und zweiten Befestigungselemente die erste und zweite geformte Länge haben.

2. Befestigungselement-Anbringungsvorrichtung nach Anspruch 1, wobei die erste Reihe von Befestigungselementen innenliegend von der zweiten Reihe von Befestigungselementen positioniert ist und jedes von einer Vielzahl der ersten Befestigungselemente einen kleineren kompressiven Raum als der kompressive Raum, der durch jedes von einer Vielzahl der zweiten Befestigungselemente definiert ist, definiert.

3. Befestigungselement-Anbringungsvorrichtung nach Anspruch 1 oder 2, wobei die erste Backe weiter eine dritte Reihe (1122a) von dritten Befestigungselementen (100a) umfasst, wobei wenigstens eines der dritten Befestigungselemente eine dritte geformte Länge (Da) hat, wenn es in eine Einzelschlaufenkonfiguration geformt ist, und die dritte Reihe von Befestigungselementen außenliegend von der zweiten Reihe von Befestigungselementen positioniert ist, wobei die zweite geformte Länge geringer als die dritte geformte Länge ist.

4. Befestigungselement-Anbringungsvorrichtung nach einem vorstehenden Anspruch, wobei jedes der Befestigungselementbeine eine Fase (112, 114) hat, um einen Nockeneffekt zu erzeugen, um eine Behinderung zu begrenzen, wenn die Beine während einer Verformung einander passieren.

5. Befestigungselement-Anbringungsvorrichtung nach einem vorstehenden Anspruch, wobei in der geformten Konfiguration eine kombinierte Dicke der Beine etwa zweimal eine Dicke des Rückenstegs ist.

6. Befestigungselement-Anbringungsvorrichtung nach Anspruch 1, wobei die ersten und zweiten Backen schwenkbar befestigt sind oder wobei wenigstens eine der ersten und zweiten Backen entlang eines im Wesentlichen linearen Weges beweglich ist, um die Ambossbacken in Annäherung zu bewegen.

7. Befestigungselement-Anbringungsvorrichtung nach einem vorstehenden Anspruch, wobei die ersten und zweiten Reihen von Befestigungselementen in einer im Wesentlichen ringförmigen Konfiguration angeordnet sind oder wobei die ersten und zweiten Reihen von Befestigungselementen in einer im Wesentlichen linearen Konfiguration angeordnet sind.

8. Befestigungselement-Anbringungsvorrichtung nach einem vorstehenden Anspruch, wobei eine Tiefe von wenigstens einer der Ambosstaschen in der erste Reihe geringer als eine Tiefe von wenigstens einer der Ambosstaschen in der zweiten Reihe ist.

9. Befestigungselement-Anbringungsvorrichtung nach einem vorstehenden Anspruch, wobei wenigstens eines der Befestigungselemente des ersten Paars von Reihen von Befestigungselementen, wenn in eine Einzelschlaufenkonfiguration geformt, eine erste Druckkraft auf Gewebe ausübt und wenigstens eines der Befestigungselemente des zweiten Paars von Reihen von Befestigungselementen, wenn in eine Einzelschlaufenkonfiguration geformt, eine zweite unterschiedliche Druckkraft auf Gewebe ausübt.

## Revendications

1. Appareil d'application d'attache chirurgicale (1000) comprenant une première mâchoire (1010) et une seconde mâchoire (1012), la première mâchoire contenant une première rangée (1122c) de premières attaches chirurgicales (100c) et une deuxième rangée (1122b) de deuxièmes attaches chirurgicales (100b), la seconde mâchoire contenant une première rangée (1206c, 2206c) de premières poches d'enclume (1204, 2204) et une seconde rangée (1206b, 2206b) de secondes poches d'enclume, les première et seconde rangées de poches d'enclume déformant les première et deuxième rangées respectives d'attaches, au moins une des premières attaches ayant une première longueur non formée (Lc) et formant une configuration ayant une première longueur formée (Dc) pour former un premier espace compressif (122c) quand déformée par la poche d'enclume, et au moins une des deuxièmes attaches ayant une deuxième longueur non formée (Lb) et formant une configuration ayant une deuxième longueur formée (Db) pour former un second espace compressif (122b) déformé par la poche d'enclume, dans lequel la première longueur non formée est inférieure à la deuxième longueur non formée et la première longueur formée est inférieure à la deuxième longueur formée ; et dans lequel chacune de l'au moins une des premières et deuxièmes attaches a une travée arrière (106) et un couple de montants (102, 104) s'étendant à partir de la travée arrière ;
**caractérisé en ce que** pendant la formation, les montants de chaque couple de montants passent l'un devant l'autre pour former une configuration de boucle simple dans laquelle les montants sont disposés dans une relation dans le sens de la largeur et l'au moins une des premières et deuxièmes attaches ont les première et deuxième longueurs formées.

2. Appareil d'application d'attache selon la revendication 1, dans lequel la première rangée d'attaches est positionnée à l'intérieur de la deuxième rangée d'attaches et chacune d'une pluralité des premières attaches définit un espace compressif plus petit que l'espace compressif défini par chacune d'une pluralité des deuxièmes attaches.

3. Appareil d'application d'attache selon les revendications 1 ou 2, la première mâchoire comprenant en outre une troisième rangée (1122a) de troisièmes attaches (100a), au moins une des troisièmes attaches ayant une troisième longueur formée (Da) lorsque formée dans une configuration de boucle simple, la troisième rangée d'attaches étant positionnée en dehors de la deuxième rangée d'attaches, la deuxième longueur formée étant inférieure à la troisième longueur formée.

4. Appareil d'application d'attache selon l'une quelconque des revendications précédentes, dans lequel chacun des montants d'attache a un chanfrein (112, 114) pour créer un effet servant de came pour limiter l'interférence lorsque les montants passent l'un devant l'autre pendant la formation.

5. Appareil d'application d'attache selon l'une quelconque des revendications précédentes, dans lequel dans la configuration formée une épaisseur combinée des montants est environ deux fois une épaisseur de la travée arrière.

6. Appareil d'application d'attache selon la revendication 1, dans lequel les première et seconde mâchoires sont attachées de manière pivotante ou dans lequel au moins une des première et seconde mâchoires est mobile le long d'un chemin sensiblement linéaire pour déplacer les mâchoires d'enclume l'une vers l'autre.

7. Appareil d'application d'attache selon l'une quelconque des revendications précédentes, dans lequel les première et deuxième rangées d'attaches sont agencées dans une configuration sensiblement annulaire ou dans lequel les première et deuxième rangées d'attaches sont agencées dans une configuration sensiblement linéaire.

8. Appareil d'application d'attache selon l'une quelconque des revendications précédentes, dans lequel une profondeur d'au moins une des poches d'enclume dans la première rangée est inférieure à une profondeur d'au moins une des poches d'enclume dans la deuxième rangée.

9. Appareil d'application d'attache selon l'une quelconque des revendications précédentes, dans lequel au moins une des attaches du premier couple de rangées d'attaches lorsque formées dans une configuration de boucle simple applique une première force compressive sur un tissu et au moins une des attaches du second couple de rangées d'attaches lorsque formées dans une configuration de boucle simple applique une seconde force compressive différente sur le tissu.
